# EUROPÄISCHE PATENTSCHRIFT

(11) **EP 3 559 662 B1**
(45) Veröffentlichungstag und Bekanntmachung des Hinweises auf die Patenterteilung: **04.10.2023**
(21) Anmeldenummer: 17835597.0
(22) Anmeldetag: 11.12.2017
(51) Int. Cl.: G01N 33/483, A61B 5/01, A61B 5/145, G16H 40/67, A61B 5/00, G01N 21/84

(54) **HAARUNTERSUCHUNGSVORRICHTUNG**
HAIR ANALYSING DEVICE
DISPOSITIF D'ANALYSE DES CHEVEUX

(30) Priorität: 20.12.2016 DE 102016225564
(43) Veröffentlichungstag der Anmeldung: 30.10.2019
(73) Patentinhaber: Henkel AG & Co. KGaA, 40589 Düsseldorf (DE)
(72) Erfinder: METTEN, Diane, 22393 Hamburg (DE); KNAPPE, Thorsten, 22869 Schenefeld (DE); PULS, Anna, 21423 Winsen (Luhe) (DE); BAYERSDÖRFER, Rolf, 22589 Hamburg (DE); ROSCHER, Katharina, 22765 Hamburg (DE)
(86) Internationale Anmeldenummer: PCT/EP2017/082238
(87) Internationale Veröffentlichungsnummer: WO 2018/114436

(56) Entgegenhaltungen:
- CN-U- 202 515 648
- US-A1- 2005 177 032
- US-A1- 2012 320 191
- US-A1- 2016 007 908
- US-A1- 2016 034 764
- US-A1- 2016 324 442

## Beschreibung

Die Erfindung betrifft eine Haaruntersuchungsvorrichtung, ein Haaruntersuchungssystem und ein Verfahren zum Bereitstellen einer Empfehlung, welche Haare eines Nutzers betrifft.

Konsumenten wissen oft wenig über den Zustand ihrer Haare und nehmen oftmals falsche Pflege- und Stylingprodukte, die die Haare beispielsweise beschweren oder schwer handhabbar machen. Eine hinreichende Bestimmung eines individuellen Haarzustands (auch als Haarstatus bezeichnet) kann zeit- und kostenaufwändig sein. Ebenso kann eine aufgrund fehlender Ausgangsdaten erfolgte kosmetische Falsch- oder Fehlbehandlung zeit- und kostenaufwändig sein.

Eine Selbstvermessung körpereigener Daten mittels so genannter Wearables (d.h. Sensoren, welche am Körper getragen werden können, entweder kurzzeitig oder für einen längeren Zeitraum) ist ein zunehmender Trend in weiten Bevölkerungskreisen. Herkömmliche verwendete Wearables eignen sich allerdings nur für ein Erfassen allgemeiner Gesundheitsparameter, z.B. Temperatur und/oder Puls, aber nicht für ein Ermitteln eines Haarzustands.

US 2016007908 stellt ein Haarprüfgerät vor, das auf dem Haar befestigt ist und haarbezogene Daten misst. US 2012320191 offenbart ein Gerät zur Analyse der Eigenschaften von Haarfasern mit Hilfe von Infrarotbildern. CN 202515648 offenbart ein Gerät, das Haut- oder Haareigenschaften mit Hilfe von LED-Technologie analysiert.

In verschiedenen Ausführungsbeispielen wird eine am oder im Haar anzuordnende Vorrichtung (auch als "Haar-Wearable" oder "Hairable" bezeichnet) bereitgestellt, welche mit einer für ein Erfassen von kosmetischen Haarparametern geeigneten, z.B. dafür ausreichenden, Sensorik ausgestattet sein kann. Unter einer Sensorik ist hier eine Gesamtheit von einem oder mehreren Sensoren zu verstehen.

Erfindungsgemäß richtet sich die Sensorik sowohl auf "interne" wie "externe" Daten richten. Anders ausgedrückt ist der mindestens eine Sensor eingerichtet, einen oder mehrere "interne" und einen oder mehrere "externe" Sensorwert/e zu erfassen.

Unter dem Begriff der "internen" Daten sind Daten zu einem kosmetischen Status, zu einem Haarzustand, z.B. einer Haargesundheit und/oder einem Kopfhautzustand, z.B. einer Kopfhautgesundheit des Konsumenten (z.B. Haardicke, Porosität der Haaroberfläche, Sebum-Gehalt), zu verstehen.

Unter dem Begriff der "externen" Daten sind Daten zu verstehen, welche eine Lebensumwelt des Konsumenten betreffen, beispielsweise eine UV-Belastung, eine Temperatur (Höhe und Schwankung), eine Luftfeuchtigkeit, usw.

Erfindungsgemäß weist die Haaruntersuchungsvorrichtung mindestens einen Sensor auf.

Der mindestens eine Sensor kann in oder an der Haaruntersuchungsvorrichtung angeordnet sein. Zumindest in Fällen, in welchen der Sensor in der Haaruntersuchungsvorrichtung angeordnet ist und der zu erfassende Sensorwert dies erforderlich macht, kann der Sensor mit einer Oberfläche des Vorrichtungskörpers in physischem und/oder optischem Kontakt sein.

In verschiedenen Ausführungsbeispielen kann die Haaruntersuchungsvorrichtung (das Hairable) in unterschiedlichen Gestaltungen aufweisen. Das Hairable kann vorzugsweise wenigstens anteilsweise in einer flexiblen, an eine jeweilige Kopfform anpassbaren Art gestaltet sein.

In verschiedenen Ausführungsbeispielen kann die Haaruntersuchungsvorrichtung Filme und/oder Folien aufweisen, welche in verschiedenen Ausführungsbeispielen mittels eines Haftmittels, z.B. eines Klebemittels oder einer Klettoberfläche, auf das Haar und ggf. auch auf die Kopfhaut aufbringbar sind oder aufgebracht werden können. In verschiedenen Ausführungsbeispielen kann der mindestens eine Sensor in dem Film oder der Folie angeordnet sein.

In verschiedenen Ausführungsbeispielen, die nicht unter den Umfang der Ansprüche fallen, kann die Haaruntersuchungsvorrichtung ein sensorbestücktes Element zur Frisurfixierung aufweisen, beispielsweise ein Haargummi, einen Haarclip, eine Haarspange, einen Haarreif oder ähnliches.

In verschiedenen Ausführungsbeispielen kann die Haaruntersuchungsvorrichtung mindestens ein fadenförmiges Anhängsel aufweisen, welches geeignet sein kann, in das Haar eingeflochten zu werden. Das mindestens eine fadenförmige Anhängsel kann an einem Vorrichtungskörper angebracht sein. Innerhalb des fadenförmigen Anhängsels kann zumindest ein Teil des mindestens einen Sensors angeordnet sein. Innerhalb des Vorrichtungskörpers kann die Schaltkreisvorrichtung angeordnet sein, die mit dem mindestens einen Sensor gekoppelt sein kann.

In verschiedenen Ausführungsbeispielen kann die Haaruntersuchungsvorrichtung fadenförmig gestaltet sein. In der fadenförmigen Haaruntersuchungsvorrichtung können der mindestens eine Sensor und die mit dem mindestens einen Sensor gekoppelte Schaltkreisvorrichtung angeordnet sein.

In verschiedenen Ausführungsbeispielen kann die fadenförmige Haaruntersuchungsvorrichtung so gestaltet sein, beispielsweise hinsichtlich Länge, Dicke und Flexibilität, dass sie in das Haar einflechtbar ist.

In verschiedenen Ausführungsbeispielen kann die fadenförmige Haaruntersuchungsvorrichtung sensorbestückte Extensions zur Haarverlängerung aufweisen, und/oder sensorbestückte, haarähnliche Implantate.

In verschiedenen Ausführungsbeispielen können mittels der Haaruntersuchungsvorrichtung erhobene Daten (d.h. die ermittelten mindestens zwei Sensorwerte und/oder basierend darauf ermittelte Daten und/oder Empfehlungen) bereitgestellt werden.

In verschiedenen Ausführungsbeispielen können lediglich die ermittelten Sensorwerte an eine Anzeigevorrichtung (z.B. ein Smartphone oder ähnliches) übermittelt und angezeigt werden.

In verschiedenen Ausführungsbeispielen können die Sensorwerte auf unterschiedliche Weise untersucht (z.B. analysiert und/oder ausgewertet) werden. Dabei kann die Untersuchung sich in verschiedenen Ausführungsbeispielen auf aktuell erhobene Daten (einen aktuellen kosmetischen Zustand (auch als kosmetischer Status bezeichnet) und/oder auf eine zeitliche Veränderung der Daten (des kosmetischen Status) beziehen.

In verschiedenen Ausführungsbeispielen kann die Analyse entweder durch die Haaruntersuchungsvorrichtung selbst, beispielsweise mittels der Schaltkreisvorrichtung.

In verschiedenen Ausführungsbeispielen, z.B. vorzugsweise, können die Daten übertragen werden auf/an eine externe Datenverarbeitungsvorrichtung (auch als externe Plattform bezeichnet), z.B. auf ein Smartphone mit App, an eine Cloud, usw. Nach der Datenübertragung auf die externe Datenverarbeitungsvorrichtung kann mittels dieser die Untersuchung der Daten ausgeführt werden, beispielsweise zum Ermitteln einer Empfehlung.

In verschiedenen Ausführungsbeispielen der Erfindung ist die Datenauswertung Grundlage einer Empfehlung, z.B. einer Produkt- und/oder Verfahrensempfehlung, z.B. kann die Empfehlung so genannte DOs (anweisende Empfehlungen) und DON`Ts (warnende Empfehlungen) aufweisen, z.B. (als Beispiel für ein DO) "Nimm das Shampoo X und style Deine Haare mit Schaumfestiger Y" oder (als Beispiel für ein DON`T) "Noch nicht waschen, versuche es heute mal mit Trockenshampoo".

In verschiedenen Ausführungsbeispielen können dem Konsumenten aktuelle, persönliche Daten hinsichtlich des kosmetischen Zustands seiner Haare bereitgestellt werden. Ein Erfassen (auch als Erhebung bezeichnet) dieser Daten durch die Haaruntersuchungsvorrichtung kann autonom und kontinuierlich erfolgen.

In verschiedenen Ausführungsbeispielen kann die autonome Datenerhebung den Verbraucher von einer Notwendigkeit entbinden, sich einem Fachmann/einer Fachfrau (z.B. Friseur/in und/oder Kosmetiker/in) oder eines externen unhandlichen Analysegeräts zu bedienen.

Eine kontinuierliche Datenerhebung der Haargesundheit kann in verschiedenen Ausführungsbeispielen eine Darstellung und Überprüfung von Verlauf und Ergebnis einer kosmetischen Behandlung erlauben.

In verschiedenen Ausführungsbeispielen kann eine Abstimmung der kosmetischen Behandlung mit individualisierten Konsumentendaten einen iterativen kosmetischen Behandlungszyklus ermöglichen, ein Ergebnis der kosmetischen Behandlung verbessern und/oder eine Motivation des Nutzers, die Behandlung fortzusetzen, erhöhen.

In verschiedenen Ausführungsbeispielen kann die Haaruntersuchungsvorrichtung, beispielsweise die mit dem Haftmittel versehene Haaruntersuchungsvorrichtung, so gestaltet sein, dass sie auch bei kurzen Haaren anwendbar sein kann.

In verschiedenen Ausführungsbeispielen kann die Haaruntersuchungsvorrichtung, beispielsweise die mit dem Haftmittel versehene Haaruntersuchungsvorrichtung, so gestaltet sein, dass sie so anordenbar ist, dass sie zumindest teilweise in Kontakt mit einer Kopfhaut des Nutzers sein kann. Damit kann ermöglicht sein, zusätzlich zum Haarzustand auch einen Kopfhautzustand des Nutzers zu ermitteln.

In verschiedenen Ausführungsbeispielen kann die Haaruntersuchungsvorrichtung so gestaltet sein, dass in einem Erfassungsbereich des mindestens einen Sensors einzelne, wenige und/oder flach ausgebreitete Haare angeordnet sein können. Dies kann ein Erfassen mancher Sensorwerte, beispielsweise von einer Haardicke o.ä., erleichtern.

In verschiedenen Ausführungsbeispielen können einem Anwender Informationen über einen Status des Haares (auch als Haarzustand bezeichnet) bereitgestellt werden, welche ferner genutzt werden können, um eine individuelle, auf den Haarzustand des Nutzers abgestimmte Empfehlung zu ermitteln, beispielsweise eine Produktempfehlung (z.B. für ein Haarpflege-, Haarfärbe- und/oder ein Haarstylingprodukt) und/oder eine Pflegeempfehlung, z.B. eine Pflegeempfehlung, welche die Haare und/oder die Kopfhaut des Nutzers betrifft.

In verschiedenen Ausführungsbeispielen kann der Haaruntersuchungsvorrichtung eine elektronische Schaltkreisvorrichtung und mindestens einen Sensor aufweisen. Die elektronische Schaltkreisvorrichtung kann mit dem mindestens einen Sensor gekoppelt sein zum Empfangen von mindestens zwei Sensorwerten In verschiedenen Ausführungsbeispielen der Erfindung ist die elektronische Schaltkreisvorrichtung eingerichtet, basierend auf dem empfangenen mindestens zwei Sensorwerten mindestens eine Empfehlung (z.B. eine Produktempfehlung für ein Haarpflegeprodukt, ein Haarfärbeprodukt oder ein Haarstylingprodukt (z.B. ein Produkt zu kurzfristigen oder dauerhaften Haarumformung), oder eine Behandlungsempfehlung, z.B. das Haar vor Sonne zu schützen, zu waschen oder mit dem Waschen noch zu warten) zu ermitteln und einem Nutzer (auch als Anwender bezeichnet) bereitzustellen.

In verschiedenen Ausführungsbeispielen kann die Empfehlung direkt mittels der Haaruntersuchungsvorrichtung ermittelt sein oder werden, d.h. die elektronische Schaltkreisvorrichtung kann eingerichtet sein, die Empfehlung selbst (auch als direkt bezeichnet) zu ermitteln. Beispielsweise kann die elektronische Schaltkreisvorrichtung eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und entweder dem Nutzer direkt bereitzustellen, oder um die Sensordaten zu verwenden, um die Empfehlung bereitzustellen. Beispielsweise können die Sensordaten mit einer Datenbank verglichen werden, welche beispielsweise empirisch gewonnen worden sein kann. In der Datenbank können einer Mehrzahl von Sensordaten Empfehlungen zugeordnet sein. Für eine Zuordnung von Empfehlungen können nicht erfindungsgemäß die Sensordaten einzeln genutzt werden, also z.B. eine Art von Sensorwert für sich genommen, z.B. eine Haardicke (z.B. für eine Empfehlung eines angesichts des einen Sensorwerts, z.B. der Haardicke, geeigneten Produkts und/oder einer geeigneten Haarbehandlung), und/oder, erfindungsgemäß, die Sensordaten können in Kombination verwendet werden, also z.B. zwei oder mehr Sensorwerte, welche kombiniert genutzt werden, z.B. eine Haardicke/Haarfarbe-Kombination, welcher angesichts der ermittelten Sensorwerte mindestens eine Empfehlung zugeordnet sein kann, z.B. ein bei der vorliegenden Kombination von Haardicke und Haarfarbe geeigneten Produkts und/oder einer geeigneten Haarbehandlung. Mittels Verwendens mehrerer Sensorwerte für das Ermitteln der mindestens einen Empfehlung kann in verschiedenen Ausführungsbeispielen eine Treffsicherheit bei dem Ermitteln der Empfehlung erhöht werden.

In verschiedenen Ausführungsbeispielen kann die elektronische Schaltkreisvorrichtung eingerichtet sein, die Empfehlung, z.B. Produkt- oder Behandlungsempfehlung, indirekt zu ermitteln. Beispielsweise kann die elektronische Schaltkreisvorrichtung (z.B. zusätzlich zu einem Speicher und einem Prozessor, z.B. einem Mikroprozessor) mit einer Datenübertragungsvorrichtung ausgerüstet sein, welche eingerichtet sein kann, die von der elektronische Schaltkreisvorrichtung empfangenen Sensordaten an eine externe Datenverarbeitungsvorrichtung, z.B. an einen Computer, z.B. eine Cloud, zu übermitteln, mittels derer, beispielsweise wie oben für das Ermitteln der Empfehlung mittels der elektronischen Schaltkreisvorrichtung beschrieben, die Empfehlung ermittelt werden kann, um die Empfehlung bereitzustellen, beispielsweise mittels Übertragens an eine Empfehlung und/oder mittels Übertragens der Empfehlung zurück an die elektronische Schaltkreisvorrichtung (z.B. mittels der Datenübertragungsvorrichtung). In verschiedenen Ausführungsbeispielen kann die Datenübertragung mehrstufig erfolgen, beispielsweise indem die Sensordaten von der Schaltkreisvorrichtung zunächst an die Anzeigevorrichtung (z.B. ein Smartphone, ein Tablet o.ä.) übermittelt werden, und die Anzeigevorrichtung die Sensordaten an die externe Datenverarbeitungsvorrichtung (z.B. die Cloud) überträgt.

In verschiedenen Ausführungsbeispielen kann das Bereitstellen der Empfehlung an den Nutzer ein Bereitstellen mittels eines Übertragens der Empfehlung an eine Anzeigevorrichtung und Anzeigen der Empfehlung aufweisen. In verschiedenen Ausführungsbeispielen kann das Bereitstellen der Empfehlung an den Nutzer ein Bereitstellen mittels eines Übertragens der Empfehlung an eine Anzeigevorrichtung und Ansagen, beispielsweise mittels eines in/an der Anzeigevorrichtung angebrachten Lautsprechers, der Empfehlung aufweisen.

Das Übertragen kann in verschiedenen Ausführungsbeispielen mittels einer drahtlosen Übertragungsvorrichtung erfolgen. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN, ZigBee, NFC, Wibree, Threald, WiMAX oder ähnlichem ermöglicht.

In verschiedenen Ausführungsbeispielen kann die Anzeigevorrichtung einen Computerbildschirm, ein Smartphone, ein Tablet, eine Smartwatch, ein iPad, einen Laptop oder ähnliches aufweisen.

In verschiedenen Ausführungsbeispielen kann ein Haaruntersuchungssystem gebildet sein von einer Haaruntersuchungsvorrichtung und der Anzeigevorrichtung.

Als Variation zu den obigen Beispielen können in verschiedenen Ausführungsbeispielen andere Kombinationen von zwei oder mehr Empfehlungen mittels der Anzeigevorrichtung bereitgestellt werden.

In verschiedenen Ausführungsbeispielen kann der mindestens eine Sensor einen Haardickensensor aufweisen. Die Haardicke kann in verschiedenen Ausführungsbeispielen einen optischen Sensor aufweisen, z.B. einen Sensor, auf welchen ein Oberflächenbereich des Vorrichtungskörpers, mit welchem Haare in Kontakt sein können, abgebildet wird, so dass eine Dicke der Haare (z.B. wie sie auf dem Oberflächenbereich erscheint) mittels einer Bildauswertung des Sensors auswertbar ist, und/oder einen Ultraschallsensor, der eingerichtet ist, auf bekannte Weise ein Ultraschallsignal auszusenden und einen von einer zu messenden Struktur (den Haaren) reflektierten Anteil des Ultraschallsignals zu untersuchen und daraus die Dicke der Haare zu ermitteln, und/oder einen Laser, der für ein an sich bekanntes Dickemessverfahren genutzt wird, z.B. für eine Interferometrie und/oder ein Triangulationsverfahren. Die gemessenen Sensorwerte können, wie oben beschrieben, entweder direkt in der Haaruntersuchungsvorrichtung ausgewertet werden, z.B. mittels der elektronischen Schaltkreisvorrichtung, oder indirekt ausgewertet werden, indem sie an eine externe Datenverarbeitungsvorrichtung übertragen werden, um dort ausgewertet zu werden, z.B. wie oben beschrieben.

In verschiedenen Ausführungsbeispielen kann der mindestens eine Sensor einen Luftfeuchtigkeitssensor aufweisen. Der mindestens eine Luftfeuchtigkeitssensor kann in physischem Kontakt sein mit einer Oberfläche des Vorrichtungskörpers, beispielsweise eine Messöffnung aufweisen. Der Kontaktbereich (z.B. die Messöffnung) kann beispielsweise auf einer einem Kopf des Nutzers abgewandten Seite des Vorrichtungskörpers angeordnet sein.

In verschiedenen Ausführungsbeispielen kann der mindestens eine Sensor einen UV-Strahlungssensor aufweisen. Der mindestens eine UV-Strahlungssensor kann in optischem Kontakt sein mit einer Oberfläche des Vorrichtungskörpers, beispielsweise auf einer einem Kopf des Nutzers abgewandten Seite des Vorrichtungskörpers. Anders ausgedrückt kann die Haaruntersuchungsvorrichtung zwischen einem Lichterfassungsbereich (z.B. einem Detektor) und einer Oberfläche der Haaruntersuchungsvorrichtung UV-lichtdurchlässig, z.B. UV-transparent gestaltet sein.

In verschiedenen Ausführungsbeispielen kann der mindestens eine Sensor ein Spektrometer und/oder eine Kamera zum Erfassen eines Absorptionsmerkmals eines zu erfassenden Stoffes, beispielsweise eines Lipids (z.B. eines Lipids, welches Teil eines Hauttalgs (Sebums) des Nutzers sein kann), einer Cysteinsäure (wobei ein Cysteinsäuregehalt als Maß für eine Haarschädigung genutzt werden kann), von Wasser oder ähnlichem aufweisen

In verschiedenen Ausführungsbeispielen kann der mindestens eine Sensor ein Thermometer aufweisen zum Erfassen einer Haartemperatur, einer Kopfhauttemperatur und/oder einer Umgebungstemperatur des Nutzers.

In verschiedenen Ausführungsbeispielen kann der mindestens eine Sensor eine Kamera aufweisen. Das von dem Sensor aufgenommene Bild kann der elektronischen Schaltkreisvorrichtung bereitgestellt werden, welche, z.B. wie oben beschrieben direkt oder indirekt, z.B. mittels Vergleichens mit Referenzdaten, eine Haarfarbe, eine Haarporösität, eine Haardichte oder andere optisch ermittelbare Haareigenschaften ermitteln kann. Die ermittelte Haareigenschaft (Haarfarbe, Haarporösität oder ähnliches) kann einbezogen werden beim Ermitteln einer Empfehlung, z.B. einer Produkt- oder Behandlungsempfehlung.

In verschiedenen Ausführungsbeispielen kann der mindestens eine Sensor andere im Prinzip bekannte Nachweisvorrichtungen aufweisen, welche wegen einer allgemeinen zunehmenden Miniaturisierung solcher Vorrichtungen in einer Haaruntersuchungsvorrichtung anordenbar sein können.

In verschiedenen Ausführungsbeispielen wird eine Haaruntersuchungsvorrichtung bereitgestellt. Die Haaruntersuchungsvorrichtung kann einen Vorrichtungskörper aufweisen, der auf mindestens einer Seite an seiner Oberfläche mit einer Haftschicht versehen ist und der eingerichtet ist, mittels der Haftschicht lösbar im oder am Haar eines Nutzers befestigt zu werden, mindestens einen im oder am Vorrichtungskörper angeordneten Sensor zum Erfassen mindestens eines Sensorwerts, und eine im oder am Vorrichtungskörper angeordnete elektronische Schaltkreisvorrichtung, wobei die elektronische Schaltkreisvorrichtung mit dem mindestens einen Sensor gekoppelt ist zum Empfangen des mindestens einen Sensorwerts, und wobei die elektronische Schaltkreisvorrichtung eingerichtet ist, den empfangenen mindestens einen Sensorwert dem Nutzer bereitzustellen.

In verschiedenen Ausführungsbeispielen wird eine Haaruntersuchungsvorrichtung bereitgestellt. Die Haaruntersuchungsvorrichtung kann einen Vorrichtungskörper aufweisen, der eingerichtet ist, im oder am Haar eines Nutzers angeordnet zu werden, wobei der Vorrichtungskörper fadenförmig ist oder einen fadenförmigen Bereich aufweist, mindestens einen Sensor zum Erfassen mindestens eines Sensorwerts, wobei der Sensor oder mindestens einer der Sensoren in dem fadenförmigen Vorrichtungskörper oder dem fadenförmigen Bereich des Vorrichtungskörpers angeordnet ist, und eine im oder am Vorrichtungskörper angeordnete elektronische Schaltkreisvorrichtung, wobei die elektronische Schaltkreisvorrichtung mit dem mindestens einen Sensor gekoppelt ist zum Empfangen des mindestens einen Sensorwerts, und wobei die elektronische Schaltkreisvorrichtung eingerichtet ist, den empfangenen mindestens einen Sensorwert dem Nutzer bereitzustellen.

In verschiedenen Ausführungsbeispielen kann die elektronische Schaltkreisvorrichtung eingerichtet sein, basierend auf dem ermittelten Sensorwert eine Empfehlung zu ermitteln und dem Nutzer bereitzustellen.

In verschiedenen Ausführungsbeispielen kann die Empfehlung mindestens eine Empfehlung ausgewählt aus der Gruppe bestehend aus Haarpflegeproduktempfehlungen, Haarfärbeproduktempfehlungen, Haarstylingproduktempfehlungen und Haarbehandlungsempfehlungen umfassen.

In verschiedenen Ausführungsbeispielen kann der Sensor mindestens einen Sensor ausgewählt aus der Gruppe bestehend aus Luftfeuchtigkeitssensoren, Temperatursensoren, UV-Strahlungssensoren, Haardickesensoren, Haarfeuchtigkeitssensoren, Schädigungsgradsensoren, und Haar- und/oder Hautfettgehaltsensoren aufweisen.

In verschiedenen Ausführungsbeispielen kann der Sensor optisch und/oder physikalisch in Kontakt sein mit einer äußeren Oberfläche des Vorrichtungskörpers.

In verschiedenen Ausführungsbeispielen kann die Haftschicht einen druckempfindlichen Klebstoff und/oder ein Klettmaterial aufweisen.

In verschiedenen Ausführungsbeispielen kann die elektronische Schaltkreisvorrichtung eine kabellose Datenaustauschvorrichtung aufweisen.

In verschiedenen Ausführungsbeispielen wird ein Haaruntersuchungssystem bereitgestellt. Das Haaruntersuchungssystem kann eine Haaruntersuchungsvorrichtung gemäß verschiedenen Ausführungsbeispielen und eine Anzeigevorrichtung aufweisen, wobei die mindestens eine Haaruntersuchungsvorrichtung eingerichtet sein kann, der Anzeigevorrichtung den Sensorwert und/oder die Empfehlung mittels der Datenaustauschvorrichtung zu übermitteln.

In verschiedenen Ausführungsbeispielen kann die Anzeigevorrichtung einen Computerbildschirm, ein Smartphone, ein Tablet, eine Smartwatch, ein iPad oder ein/en Laptop aufweisen oder sein.

In verschiedenen Ausführungsbeispielen wird ein Verfahren zum Bereitstellen einer Haarzustandsinformation bereitgestellt. Das Verfahren kann ein Anordnen einer Haaruntersuchungsvorrichtung gemäß verschiedenen Ausführungsbeispielen an Haaren eines Nutzers aufweisen, ein Erfassen von mindestens einem Sensorwert des mindestens einen Sensors der Haaruntersuchungsvorrichtung, und ein Bereitstellen des mindestens einen Sensorwerts an den Nutzer.

In verschiedenen Ausführungsbeispielen kann das Verfahren ferner ein Ermitteln mindestens einer Empfehlung basierend auf dem mindestens einen Sensorwert aufweisen.

In verschiedenen Ausführungsbeispielen kann die Empfehlung mindestens eine Empfehlung ausgewählt aus der Gruppe bestehend aus Haarpflegeproduktempfehlungen, Haarstylingproduktempfehlungen, Haarfärbeproduktempfehlung und Haarbehandlungsempfehlungen umfassen.

In verschiedenen Ausführungsbeispielen kann das Verfahren ferner ein Auslösen einer Bestellung eines empfohlenen Haarpflegeprodukts, Haarstylingprodukts und/oder Haarfärbeprodukts aufweisen.

In verschiedenen Ausführungsbeispielen kann das Anordnen der Haaruntersuchungsvorrichtung ein Anordnen für einen, verglichen mit einer für ein einzelnes Erfassen des mindestens einen Sensorwerts benötigten Zeitdauer, langen Zeitraum aufweisen, und das Erfassen des mindestens einen Sensorwerts kann während des Zeitraums wiederholt oder kontinuierlich vorgenommen werden.

In verschiedenen Ausführungsbeispielen kann das Verfahren ferner ein Übermitteln des mindestens einen Sensorwerts an eine externe Datenverarbeitungsvorrichtung und ein Empfangen der mindestens einen Empfehlung von der externen Datenverarbeitungsvorrichtung aufweisen, wobei das Ermitteln der mindestens einen Empfehlung basierend auf dem mindestens einen Sensorwert mittels der externen Datenverarbeitungsvorrichtung erfolgen kann.

Ausführungsbeispiele der Erfindung sind in den Figuren dargestellt und werden im Folgenden näher erläutert.

### Es zeigen

Figuren 1A bis 1D schematische Darstellungen von Haaruntersuchungsvorrichtungen gemäß verschiedenen Ausführungsbeispielen;
Figur 2A eine schematische Darstellung eines Haaruntersuchungssystems gemäß verschiedenen Ausführungsbeispielen in Verbindung mit einer externen Datenverarbeitungsvorrichtung;
Figur 2B eine schematische Darstellung eines Haaruntersuchungssystems gemäß verschiedenen Ausführungsbeispielen;
Figur 3 eine schematische Darstellung einer Anwendung eines Haaruntersuchungssystems gemäß verschiedenen Ausführungsbeispielen;
Figur 4 ein Flussdiagramm eines Verfahrens zum Bereitstellen einer Haarzustandsinformation gemäß verschiedenen Ausführungsbeispielen; und
Figur 5 ein Flussdiagramm eines Verfahrens zum Bereitstellen einer Haarzustandsinformation gemäß verschiedenen Ausführungsbeispielen, wobei nur die Ausführungsbeispiele, bei denen mindestens zwei Sensorwerte erfasst werden, auch Ausführungsbeispiele der Erfindung sind

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

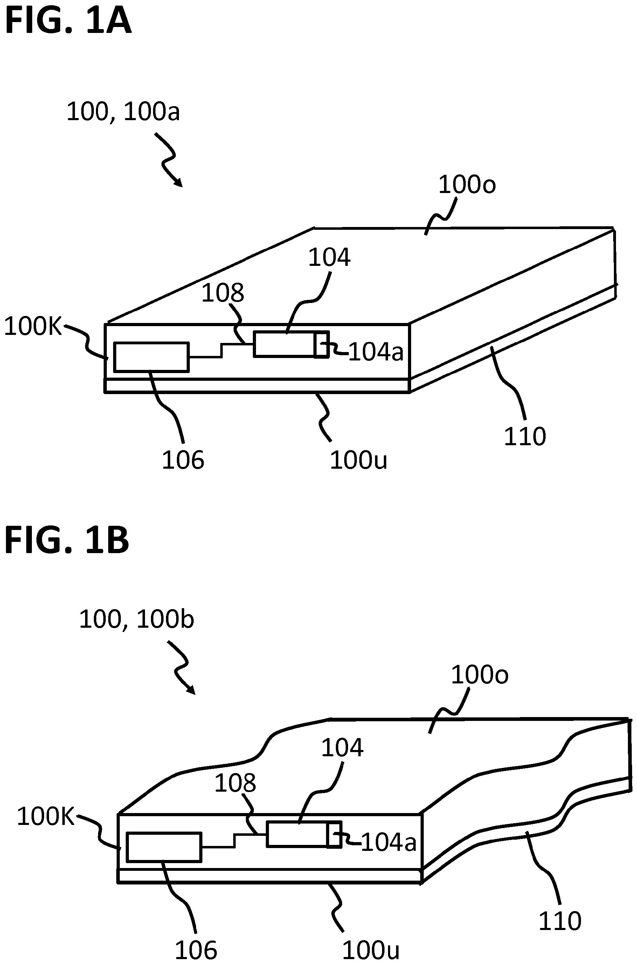

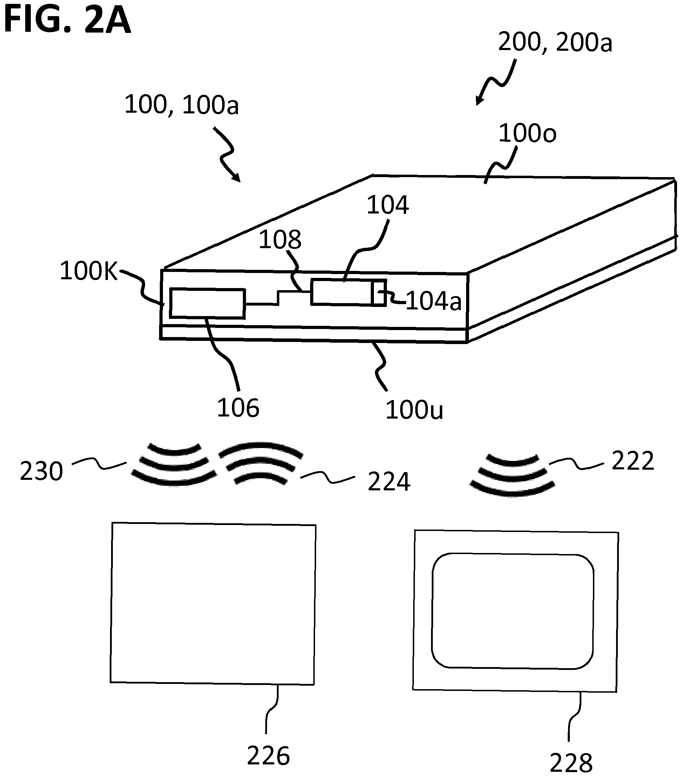

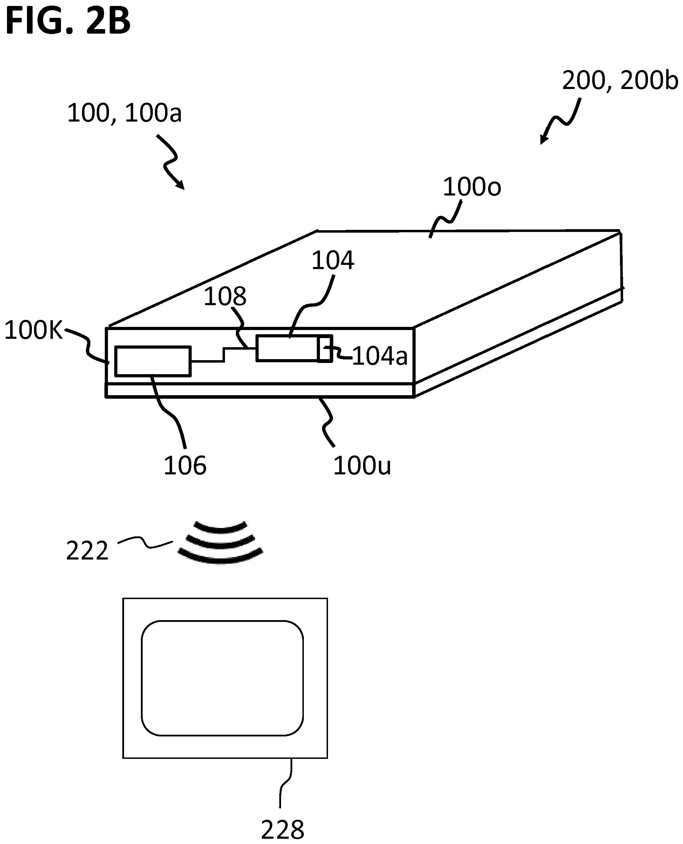

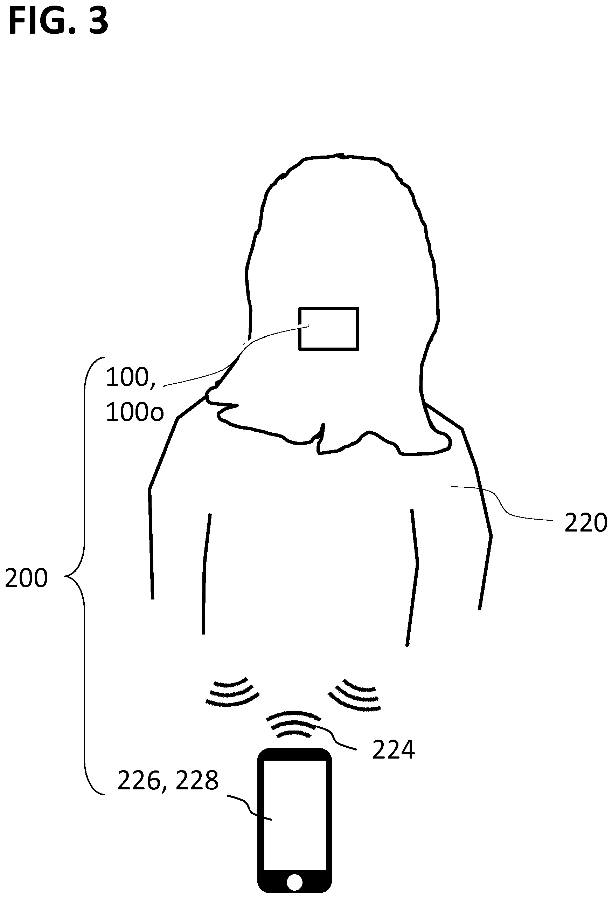

FIG. 1A bis FIG. 1D zeigen schematische Darstellungen von Haaruntersuchungsvorrichtungen 100 gemäß verschiedenen Ausführungsbeispielen, FIG. 2A zeigt eine schematische Darstellung eines Haaruntersuchungssystems 200, 200a gemäß verschiedenen Ausführungsbeispielen in Verbindung mit einer externen Datenverarbeitungsvorrichtung 226, FIG. 2B zeigt eine schematische Darstellung eines Haaruntersuchungssystems 200, 200b gemäß verschiedenen Ausführungsbeispielen, und FIG. 3 zeigt eine schematische Darstellung einer Anwendung eines Haaruntersuchungssystems 200 gemäß verschiedenen Ausführungsbeispielen.

In verschiedenen Ausführungsbeispielen wird ein Haaruntersuchungsvorrichtung 100 (verschiedene Ausführungsbeispiele sind als 100a, 100b, 100c bzw. 100d gekennzeichnet) bereitgestellt.

In verschiedenen Ausführungsbeispielen kann die Haaruntersuchungsvorrichtung 100 einen Vorrichtungskörper 100K aufweisen. Der Vorrichtungskörper 100K kann so eingerichtet sein, dass er im oder am Haar 220H eines Nutzers 220 anordenbar ist.

In verschiedenen Ausführungsbeispielen außerhalb des Gegenstands der Ansprüche (nicht dargestellt) kann der Vorrichtungskörper 100K so gestaltet sein, dass er mittels seiner mechanischen Gestaltung und/oder seiner elastischen Eigenschaften im Haar 220H des Nutzers 220 anordenbar ist, beispielsweise als Haargummi, Haarreif, dekorativer Haarkamm, Haarklemme o.ä. Der Vorrichtungskörper 100K kann aus einem festen Material, z.B. Kunststoff oder Metall, gebildet sein oder ein solches Material aufweisen.

Wie in FIG. 1A dargestellt ist, kann der Vorrichtungskörper 100K auf mindestens einer Seite 100u an seiner Oberfläche mit einer Haftschicht 110 versehen sein, mittels derer die Haaruntersuchungsvorrichtung 100 im/am Haar des Nutzers 220 anordenbar sein kann. Die Haftschicht 110 kann den Vorrichtungskörper 100K auf der Seite 100u vollständig oder teilweise bedecken.

In verschiedenen Ausführungsbeispielen kann die Haaruntersuchungsvorrichtung 100 mittels der Haftschicht 110 lösbar im oder am Haar eines Nutzers befestigt sein oder werden. Die Haftschicht 110 kann in verschiedenen Ausführungsbeispielen einen druckempfindlichen Klebstoff und/oder ein Klettmaterial aufweisen.

Wie in FIG. 1B für die Haaruntersuchungsvorrichtung 100b anhand des gewellt dargestellten Vorrichtungskörpers 100K angedeutet sein soll, kann der Vorrichtungskörper 100K in verschiedenen Ausführungsbeispielen flexibel sein. Damit kann es ermöglicht sein, die Haaruntersuchungsvorrichtung 100b beim Anordnen im/am Haar 220H des Nutzers 220 an eine Kopfform und/oder eine Frisurdes Nutzers anzupassen. Beispielsweise kann der Vorrichtungskörper 100K als Folie oder Film gebildet sein.

In verschiedenen Ausführungsbeispielen kann eine Oberseite 100o des Vorrichtungskörpers 100K dekorativ gestaltet sein.

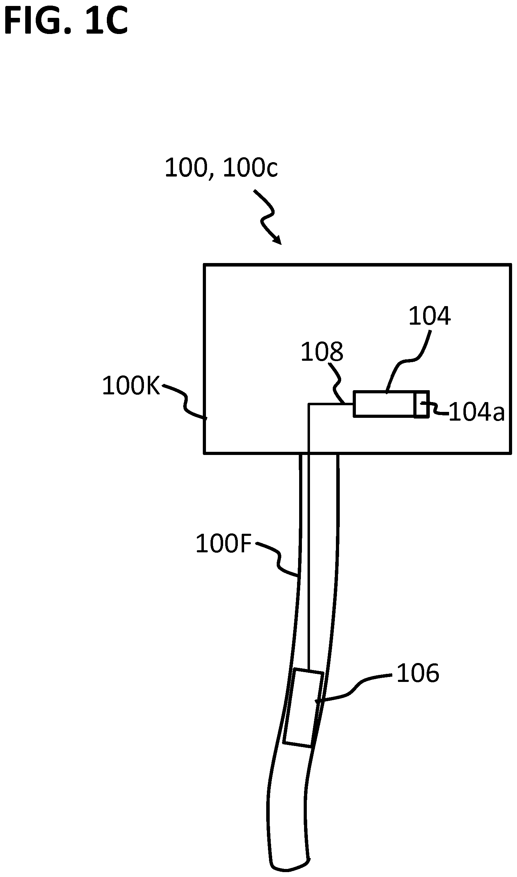

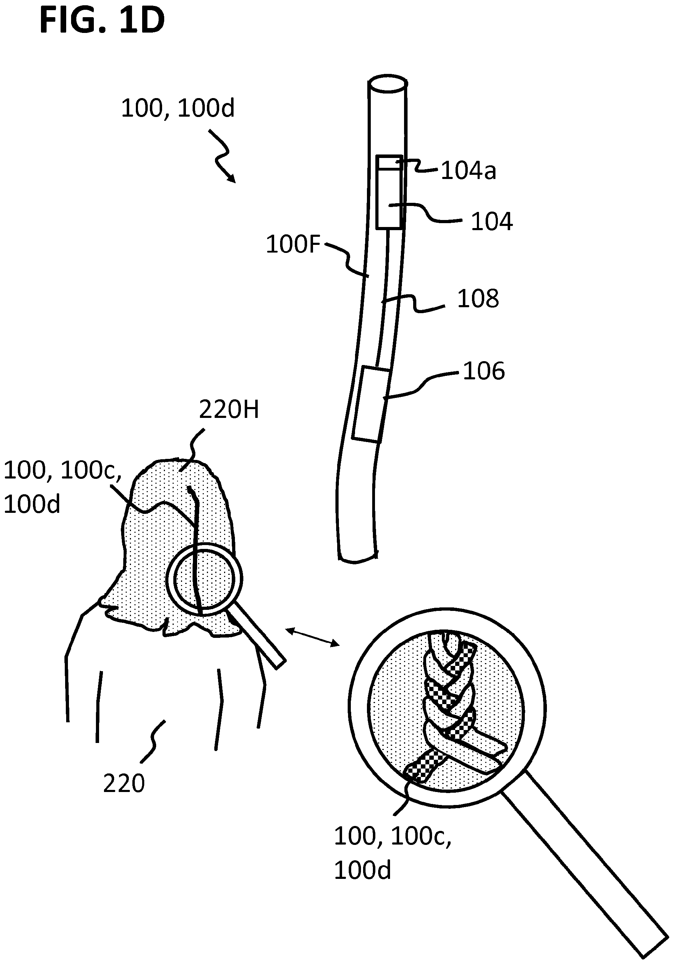

Wie in FIG. 1C und FIG. 1D dargestellt ist, kann in verschiedenen Ausführungsbeispielen der Vorrichtungskörper 100K einen fadenförmigen Bereich 100F aufweisen (FIG. 1C) oder aus einem solchen bestehen (FIG. 1D).

Der fadenförmige Bereich 100F kann in verschiedenen Ausführungsbeispielen so gestaltet sein, z.B. hinsichtlich seiner Länge, Dicke und/oder Flexibilität, dass er in das Haar 220H des Nutzers 220 einflechtbar ist, wie in FIG. 1D unten dargestellt ist. In verschiedenen Ausführungsbeispielen kann die Haaruntersuchungsvorrichtung 100 bereits durch das Einflechten des fadenförmigen Bereichs 100F am Haar 220H befestigt werden bzw. befestigbar sein. In anderen Ausführungsbeispielen kann die Haaruntersuchungsvorrichtung 100 außerdem noch mittels anderer Befestigungsmittel am Haar 220H befestigt werden bzw. befestigbar sein, z.B. mittels der Haftschicht 110, mittels einer Klemmvorrichtung, mittels eines als Kamm ausgebildeten Teils der Haaruntersuchungsvorrichtung, oder ähnlichem.

In verschiedenen Ausführungsbeispielen kann die Haaruntersuchungsvorrichtung 100 mindestens einen im oder am Vorrichtungskörper 100K angeordneten Sensor 106 zum Erfassen mindestens eines Sensorwerts aufweisen.

Der mindestens eine Sensor 106 kann in verschiedenen Ausführungsbeispielen wie oben beschrieben genutzt werden zum Ermitteln von Sensorwerten und zum Ermitteln einer Empfehlung.

In verschiedenen Ausführungsbeispielen kann der mindestens eine Sensor 106 derart in dem Vorrichtungskörper 100K angeordnet sein, dass er mit dem Haar 220H und/oder einer Umgebung des Haars 220H in Wechselwirkung treten kann.

In verschiedenen Ausführungsbeispielen, in welchen der Vorrichtungskörper 100K einen fadenförmigen Bereich 100F aufweist oder daraus besteht, kann mindestens ein Teil des mindestens einen Sensors 106 im fadenförmigen Bereich 100F angeordnet sein. Beispielsweise kann der Teil des Sensors 106 oder der Sensor 106 derart im fadenförmigen Bereich 106F angeordnet sein, dass der Sensormesswert an oder nahe einer Stelle der Haare ermittelt wird, an welcher sich der Sensor 106 oder der Teil des Sensors 106 befindet. In verschiedenen Ausführungsbeispielen können mehrere (z.B. gleichartige oder unterschiedliche) Sensoren 106 im fadenförmigen Bereich 106F angeordnet sein, welche Sensorwerte von unterschiedlichen Bereichen, z.B. entlang einer Haarlänge, bereitstellen können.

In verschiedenen Ausführungsbeispielen kann der mindestens eine Sensor 106 jegliche, beispielsweise auch im Prinzip bekannte, Nachweisvorrichtung aufweisen, welche geeignet ist, Sensordaten bereitzustellen, die Auskunft zu geben geeignet sind über einen Haarzustand eines Nutzers und/oder eine Umgebung, welcher das Haar 220H des Nutzers 220 ausgesetzt sein kann, und welche wegen einer Miniaturisierung solcher Vorrichtungen in einer Haaruntersuchungsvorrichtung 100 wie hierin beschrieben (beispielsweise in einer Haaruntersuchungsvorrichtung, die als Haftfilm, Haarclip, Haargummi, Haarreif, Flechtfaden oder ähnliches gebildet ist) anordenbar sein können.

In verschiedenen Ausführungsbeispielen kann der mindestens eine Sensor 106 wie oben beschrieben einen Haardickensensor aufweisen, z.B. einen optischen Sensor und/oder einen Ultraschallsensor und/oder einen Laser.

In verschiedenen Ausführungsbeispielen kann der mindestens eine Sensor 106 wie oben beschrieben ein Spektrometer und/oder eine Kamera zum Erfassen eines Absorptionsmerkmals eines zu erfassenden Stoffes, beispielsweise eines Lipids (z.B. eines Lipids, welches Teil eines Hauttalgs (Sebums) des Nutzers sein kann), einer Cysteinsäure, von Wasser oder ähnlichem aufweisen.

In verschiedenen Ausführungsbeispielen kann der mindestens eine Sensor 106 wie oben beschrieben ein Thermometer aufweisen zum Erfassen einer Haartemperatur, einer Kopfhauttemperatur und/oder einer Umgebungstemperatur des Nutzers.

In verschiedenen Ausführungsbeispielen kann der mindestens eine Sensor 106 eine Kamera aufweisen, wie oben beschrieben, beispielsweise als Haarfarbsensor und/oder zum Ermitteln einer Haarporösität.

In verschiedenen Ausführungsbeispielen kann der mindestens eine Sensor 106, wie oben beschrieben, einen Luftfeuchtigkeitssensor und/oder einen UV-Strahlungssensor aufweisen.

In verschiedenen Ausführungsbeispielen kann die Haaruntersuchungsvorrichtung 100 eine im oder am Vorrichtungskörper 100K angeordnete elektronische Schaltkreisvorrichtung 104 aufweisen.

Die elektronische Schaltkreisvorrichtung 104 kann in verschiedenen Ausführungsbeispielen mit dem mindestens einen Sensor 106 gekoppelt sein, beispielsweise mittels einer Verbindung 108, zum Empfangen der mindestens zwei Sensorwerte.

In verschiedenen Ausführungsbeispielen, in welchen die Haaruntersuchungsvorrichtung aus einem fadenförmigen Bereich 100F besteht, kann die elektronische Schaltkreisvorrichtung 104, wie in FIG. 1D beispielhaft dargestellt, in dem fadenförmigen Bereich 100F angeordnet sein.

In verschiedenen Ausführungsbeispielen, in welchen die Haaruntersuchungsvorrichtung aus den fadenförmigen Bereich 100F aufweist, kann die elektronische Schaltkreisvorrichtung 104, wie in FIG. 1C dargestellt, in dem anders (z.B. filmartig) geformten Bereich angeordnet sein, oder die elektronische Schaltkreisvorrichtung 104 kann in dem fadenförmigen Bereich 100F angeordnet sein (nicht dargestellt).

Ferner kann die elektronische Schaltkreisvorrichtung 104 in verschiedenen Ausführungsbeispielen eingerichtet sein, die empfangenen mindestens zwei Sensorwerte dem Nutzer 220 bereitzustellen.

In verschiedenen Ausführungsbeispielen kann die elektronische Schaltkreisvorrichtung 104 eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und entweder dem Nutzer direkt bereitzustellen, oder um die Sensordaten zu verwenden, um die Empfehlung bereitzustellen.

Die elektronische Schaltkreisvorrichtung 104 kann in verschiedenen Ausführungsbeispielen gekoppelt sein, z.B. mittels einer Verbindung 108, mit dem mindestens einen Sensor 106. Beim Vorhandensein einer Mehrzahl von Sensoren 106 kann die Schaltkreisvorrichtung 104 eine eigene Kopplung zu jedem der Sensoren 106 aufweisen. Die Kopplung kann in verschiedenen Ausführungsbeispielen eine elektrisch leitende Verbindung, eine (Glas-)faserverbindung und/oder eine kabellose Verbindung aufweisen oder sein. Die elektronische Schaltkreisvorrichtung 104 kann zum Empfangen der mindestens zwei Sensorwerte von dem mindestens einen Sensor 106 eingerichtet sein.

Die elektronische Schaltkreisvorrichtung 104 kann in verschiedenen Ausführungsbeispielen eingerichtet sein, basierend auf den empfangenen mindestens zwei Sensorwerten mindestens eine

### Empfehlung zu ermitteln und einem Nutzer 220 bereitzustellen.

Die erfassten (gemessenen) Sensorwerte können, wie oben beschrieben, entweder direkt in der Haaruntersuchungsvorrichtung 100 ausgewertet werden, z.B. mittels der elektronischen Schaltkreisvorrichtung 104, oder indirekt ausgewertet werden, indem sie an eine externe Datenverarbeitungsvorrichtung übertragen werden, um dort ausgewertet zu werden, z.B. wie oben beschrieben. Dabei können die Sensordaten oder Teile der Sensordaten in verschiedenen Ausführungsbeispielen auf im Prinzip bekannte Weise ausgewertet werden, beispielsweise mittels einer Bildverarbeitungssoftware, z.B. zum Ermitteln der Haarfarbe, der Haardicke, zum Bestimmen der Haarporösität o.ä., und/oder im Vergleich mit (z.B. empirisch gewonnenen) Datenbankeinträgen.

In verschiedenen Ausführungsbeispielen kann die ermittelte Haardicke, Haarfarbe, Haarporösität, Haar-/Hauttemperatur, der ermittelte Haarschädigungsgrad (z.B. anhand einer Cysteinsäurekonzentration), die ermittelte Haar-, Kopfhaut- und/oder Umgebungstemperatur oder eine daraus ermittelte Temperaturschwankung, die ermittelte Luftfeuchtigkeit, UV-Strahlung und/oder ein oder mehrere weitere direkt ermittelte Sensorwerte und/oder anhand der Sensorwerte ermittelte Parameter einbezogen werden bei einem Ermitteln einer Empfehlung, z.B. einer Produkt- oder Behandlungsempfehlung.

In verschiedenen Ausführungsbeispielen kann das Anordnen der Haaruntersuchungsvorrichtung 100 ein Anordnen für einen, verglichen mit einer für ein einzelnes Erfassen des mindestens einen Sensorwerts benötigten Zeitdauer, langen Zeitraum aufweisen. Die Haaruntersuchungsvorrichtung 100 kann beispielsweise für einen ein- oder mehrstündigen Zeitraum belassen werden, beispielsweise einen großen Teil des Tages.

In verschiedenen Ausführungsbeispielen kann das Erfassen der mindestens zwei Sensorwerte während des Zeitraums, in dem die Haaruntersuchungsvorrichtung 100 im oder am Haar 220H angeordnet ist, wiederholt oder kontinuierlich vorgenommen werden. Damit kann beispielsweise ermöglicht werden, während eines Tages, z.B. eines typisch ablaufenden Tages, die Haaruntersuchung wiederholt oder kontinuierlich durchzuführen, so dass die den Haarzustand und/oder äußere Einflüsse charakterisierenden Sensorwerte für eine beispielsweise regelmäßig wiederkehrende Situation erfasst werden können. Dementsprechend können Produkt- und/oder Behandlungsempfehlungen angepasst werden an eine voraussichtlich wieder eintretende Situation. Beispielsweise kann bei voraussichtlichem (z.B. weil wiederholt auftretendem) Aufenthalt in Räumen mit z.B. niedriger Luftfeuchtigkeit ein feuchtigkeitsspendendes Haarpflegeprodukt empfohlen werden, entsprechend z.B. bei voraussichtlichem (längerem) Aufenthalt im Freien ein Haarpflegeprodukt mit UV-Schutz, usw.

Wie oben beschrieben kann die elektronische Schaltkreisvorrichtung 104 in verschiedenen Ausführungsbeispielen eingerichtet sein, basierend auf den empfangenen mindestens zwei Sensorwerten mindestens eine Empfehlung zu ermitteln und einem Nutzer bereitzustellen, indem sie wie z.B. in FIG. 2B dargestellt, die Empfehlung selbst (direkt) ermittelt, beispielsweise wie oben beschrieben, z.B. mittels Vergleichens mit in einem Speicher der elektronischen Schaltkreisvorrichtung 104 gespeicherten Referenzwerten, -wertebereichen und/oder Datenbanken, wobei den Referenzwerten, -wertebereichen bzw. Datenbanken jeweils mindestens eine Empfehlung zugeordnet sein kann.

Wie oben beschrieben kann die elektronische Schaltkreisvorrichtung 104 in verschiedenen Ausführungsbeispielen eingerichtet sein, basierend auf den empfangenen mindestens zwei Sensorwerten mindestens eine Empfehlung zu ermitteln und einem Nutzer bereitzustellen, indem sie, wie z.B. in FIG. 2A dargestellt, die Empfehlung indirekt ermittelt, beispielsweise wie oben beschrieben, z.B. mittels Übermittelns (veranschaulicht als Übertragungssignal 230) des mindestens einen Sensorwerts an eine externe Datenverarbeitungsvorrichtung 226, z.B. einen externen Computer, z.B. eine Cloud, Vergleichens mit in einem Speicher der externen Datenverarbeitungsvorrichtung 226 gespeicherten Referenzwerten, -wertebereichen und/oder Datenbanken, wobei den Referenzwerten, -wertebereichen bzw. Datenbanken jeweils mindestens eine Empfehlung zugeordnet sein kann. Die Haaruntersuchungsvorrichtung 100 kann eingerichtet sein, die von der externen Datenverarbeitungsvorrichtung 226 ermittelte Empfehlung zu empfangen, d.h. die ermittelte Empfehlung kann der Haaruntersuchungsvorrichtung 100 übermittelt werden (veranschaulicht als Übertragungssignal 224).

In verschiedenen Ausführungsbeispielen kann ein Haaruntersuchungssystem 200 bereitgestellt sein, wie dies in FIG. 2A, FIG. 2B und FIG. 3 beispielhaft gezeigt ist. Das Haaruntersuchungssystem kann eine Haaruntersuchungsvorrichtung 100 gemäß verschiedenen Ausführungsbeispielen und eine Anzeigevorrichtung 228 aufweisen.

Zum Übermitteln der mindestens zwei Sensorwerte und/oder der mindestens einen Empfehlung an die Anzeigevorrichtung 228 und/oder zum Übermitteln der mindestens zwei Sensorwerte an die externe Datenverarbeitungsvorrichtung 226 und/oder zum Empfangen der mindestens einen Empfehlung von der externen Datenverarbeitungsvorrichtung 226 kann die Haaruntersuchungsvorrichtung 100 mit einer Übertragungsvorrichtung 104a für eine drahtlose Datenübertragung ausgestattet sein, beispielsweise mit einem Chip oder Tag, der die drahtlose Datenübertragung, z.B. mittels Bluetooth, WLAN, ZigBee, NFC, Wibree, Threald, WiMAX oder ähnlichem ermöglicht. In verschiedenen Ausführungsbeispielen kann die Übertragungsvorrichtung 104a Teil der elektronischen Schaltkreisvorrichtung 104 sein.

Wie in FIG. 3 dargestellt ist, kann die Anzeigevorrichtung 228 in verschiedenen Ausführungsbeispielen eine integrierte Einheit mit der Datenverarbeitungsvorrichtung 226 bilden, beispielsweise in Form eines Smartphones, Tablets, Laptops, Smartwatches, iPads, o.ä.

In verschiedenen Ausführungsbeispielen kann die Haaruntersuchungsvorrichtung eine integrierte Energieversorgung (nicht dargestellt) aufweisen, beispielsweise wiederaufladbare Batterien o.ä., welche eine Betriebsspannung für die elektronische Schaltkreisvorrichtung 104, die Übertragungsvorrichtung 104a und den mindestens einen Sensor 106 bereitstellen kann. Die Energieversorgung kann in verschiedenen Ausführungsbeispielen auswechselbar gestaltet sein. In verschiedenen Ausführungsbeispielen kann die Energieversorgung (z.B. wasserdicht) verschlossen, z.B. eingegossen, in der Haaruntersuchungsvorrichtung 100 angeordnet sein. In dem Fall kann die Energieversorgung beispielsweise induktiv aufladbar sein.

In verschiedenen Ausführungsbeispielen kann die Haaruntersuchungsvorrichtung 100 in Verbindung mit der Anzeigevorrichtung 228 und/oder der externen Datenverarbeitungsvorrichtung 226 als ein Haaruntersuchungssystem 200 betrachtet werden.

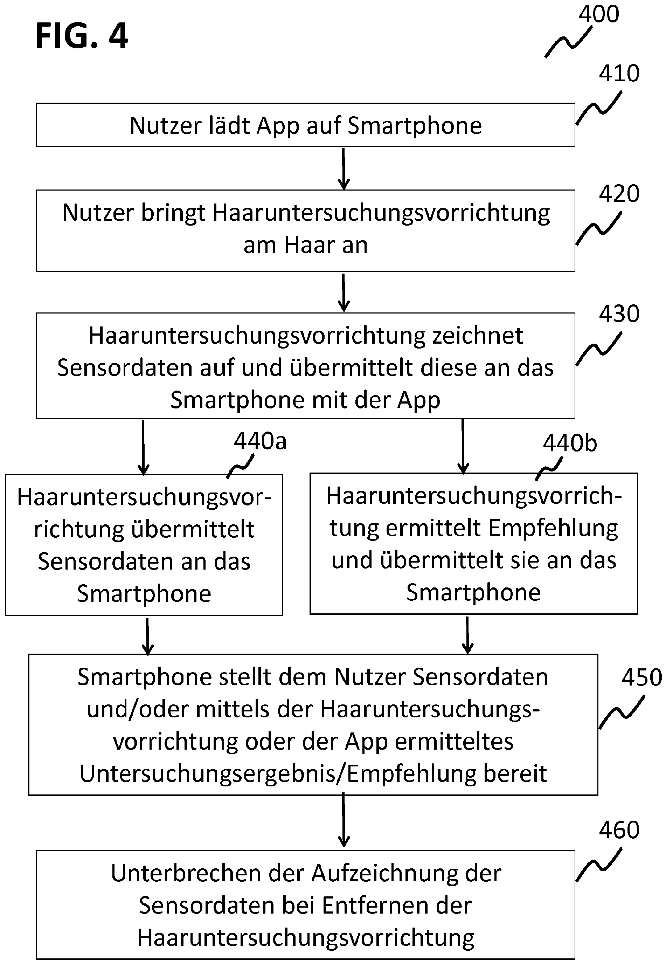

FIG. 4 zeigt ein Flussdiagramm 400 eines Verfahrens zum Bereitstellen einer Haarzustandsinformation gemäß verschiedenen Ausführungsbeispielen.

Ein Nutzer kann eine Haaruntersuchungsvorrichtung (ein Hairable) erwerben, da er wissen möchte, ob er die richtigen Pflege-, Färbe- und/oder Styling Produkte für sein Haar verwendet.

Der Nutzer kann in verschiedenen Ausführungsbeispielen eine zum Hairable gehörige mobile Applikation (App) auf sein Smartphone herunterladen (z.B. über einen App Store) (in 410).

In verschiedenen Ausführungsbeispielen kann es nötig und/oder sinnvoll sein, sich mit seinem Namen und seiner E-Mail-Adresse zu registrieren. In verschiedenen Ausführungsbeispielen kann es nötig und/oder sinnvoll sein, dass der Nutzer weitere Informationen über sich, wie beispielsweise Adresse oder Bankverbindung, hinterlegt.

In verschiedenen Ausführungsbeispielen kann der Nutzer die Haaruntersuchungsvorrichtung (das Hairable), z.B. in Form einer Klebefolie, im oder am Haar anbringen (in 420).

In verschiedenen Ausführungsbeispielen kann der Nutzer das Hairable tagsüber tragen. Während des Tragens kann die Haaruntersuchungsvorrichtung Sensordaten aufzeichnen, beispielsweise kontinuierlich (in 430).

Die Haaruntersuchungsvorrichtung kann in verschiedenen Ausführungsbeispielen die Außenumgebung (UV-Strahlung, Luftfeuchtigkeit, usw.) und die Haarstruktur und die Kopfhaut des Nutzers analysieren. Diese Daten können sich kontinuierlich mit der App synchronisieren, d.h. die mittels der Haaruntersuchungsvorrichtung ermittelten Untersuchungsergebnisse und/oder Empfehlungen können dem Smartphone übermittelt werden (in 440b).

In anderen Ausführungsbeispielen kann die Haaruntersuchungsvorrichtung die Sensordaten dem Smartphone übermitteln (in 440a), und das Smartphone kann die Untersuchungsergebnisse und/oder Empfehlungen ermitteln (in 450). In verschiedenen Ausführungsbeispielen kann das Smartphone dem Nutzer die Sensordaten und/oder das mittels der Haaruntersuchungsvorrichtung oder der App ermittelte Untersuchungsergebnis bzw. die mindestens eine Empfehlung bereitstellen (in 450).

Am Ende eines Untersuchungszeitraums, z.B. abends, kann der Nutzer das Hairable aus dem Haar entfernen, und die Datenaufzeichnung wird unterbrochen (in 460).

In verschiedenen Ausführungsbeispielen konnten schon genug Daten gesammelt werden, damit der Nutzer über die Smartphone App eine Analyse erhält, die ihn über möglich negative Außeneinflüsse und seine Haarbeschaffenheit informieren.

Basierend auf diesen Daten kann die App auch eine Empfehlung für Pflege-, Färbe- und Stylingprodukte geben, und/oder Anwendungshinweise (in 450).

In verschiedenen Ausführungsbeispielen ist es nicht nötig, dass das Tragen des Hairable täglich stattfindet, jedoch können sich die Datenanalysen und Empfehlungen verbessern, je mehr Daten gesammelt werden können.

In verschiedenen Ausführungsbeispielen können die Empfehlung für Pflege-, Färbe- und Stylingprodukte und/oder die Anwendungshinweise sich auf eine chemische Zusammensetzung der empfohlenen und nicht zu empfehlenden Pflege-, Färbe- und Stylingprodukte beziehen, beispielsweise in Form so genannter DOs (Empfehlungen) und DON'Ts (Warnungen). Ein Beispiel dafür wäre "Nimm (k)ein wachshaltiges Produkt".

Je nach Haardicke und Haardichte kann dem Nutzer in verschiedenen Ausführungsbeispielen ein geeignetes Stylingprodukt empfohlen werden. Beispielsweise kann es unvorteilhaft sein, bei dünnem, lichtem Haar ein Styling-Wachs anzuwenden, da dieses bei einem solchen Haartyp schnell fettig und ungepflegt wirkt. Unter die Kategorie "Wachs" fallen Produkte, die sich durch einen hohen Anteil an Wachskomponenten, Vaselinen, Emulgatoren und Ölen bzw. öligen oder ölhaltigen Komponenten auszeichnen. Ebenfalls nicht zu empfehlen bei dünnem lichtem Haar können Emulsionen sein, die sich ebenfalls durch einen hohen Anteil eben beschriebener Rohstoffe auszeichnen können.

Je nach Lockenzustand (eher Locken oder eher Wellen) kann dem Nutzer in verschiedenen Ausführungsbeispielen ein Produkt empfohlen werden, welches sein natürliches Lockenbild entweder unterstützt, oder im Falle von Wellen auch verstärken (auch als pushen bezeichnet) und stabilisieren kann. Bestimmte Polymere, wie bspw. Styleze CC10 (VP/DMAPA Acrylates Copolymer), Mirustyle CP (Polyquaternium-72) können hier besonders hilfreich sein. Allerdings können auch Standardpolymere, wie PVP, PVP/VA, etc. durchaus geeignet sein, den Halt der Locken zu unterstützen, und/oder weitere Polymere, deren Lockenhalte- bzw. - erzeugungsvermögen zum Zeitpunkt der Veröffentlichung dem Fachmann bekannt sind.

Locken benötigen meist viel Pflege, so dass auch bei lockigem Haar eine pflegende Emulsion/Lotion vorteilhaft sein kann. Weiterhin können Mousses angewandt werden, um Bounce und Sprungkraft zu unterstützen und die Locken zu definieren. In verschiedenen Ausführungsbeispielen können die oben für die Lockendefinition genannten Polymere genutzt werden. Die Lockendefinition kann ebenfalls mit einem leichten Wachs gelingen, wohingegen ein (schweres) Wachs bei Wellen eher beschwerend wirkt und dem Konsumenten vermutlich nicht helfen kann sein Stylingergebnis über einen längeren Zeitraum aufrechtzuerhalten. Zusätzliche Unterstützung können in verschiedenen Ausführungsbeispielen allerdings auch hier Polymere (beispielsweise wie oben genannt) liefern, um das Ergebnis und die Halteleistung zu verlängern.

Auch bei langen Haaren kann in verschiedenen Ausführungsbeispielen eine Anwendung eines Wachses oder Geles eher unüblich sein. In diesem Fall kann dem Nutzer empfohlen werden, auf leichtere Konsistenzen, wie z.B. Blow-Dry Sprays, Mousses, Lotionen o.ä. zurückgreifen, um ihren Look zu unterstützen. Besonders sprühbare oder auch niedrigviskose pumpbare Produkte können hier gut geeignet sein, da sich diese besonders leicht in der gesamten Haarlänge verteilen lassen. Je nach gewünschtem Styling Effekt kann es auch hier unterschiedliche Polymertypen geben, die zum Einsatz kommen können (z.B. PVP, PVP/VA, Chitosan, Polyquaternium-Typen, usw.).

In verschiedenen Ausführungsbeispielen kann eine Wahl des richtigen Produktes meist mit dem gewünschten Stylingergebnis zusammenhängen. So kann beispielsweise ein Blow-Dry Spray oder ein Mousse dafür vorgesehen sein, unterstützend beim Föhnen zu wirken, und kann zugleich pflegende Stylingkomponenten enthalten, die ein Arbeiten mit Kamm und Bürste im feuchten Haar zulassen (bspw. Polyquaternium-4,11,46, usw., Chitosan, Polyacrylamidopropyltrimonium Chloride, usw.).

Ein Haarspray kann dafür vorgesehen sein, die finale Frisur dann noch zu fixieren und zudem möglichst davor schützen, dass äußere Einflüsse die Frisur gefährden. Ein Beispiel für einen solchen äußeren Einfluss wäre der Einfluss hoher Luftfeuchtigkeit. Bestimmte Polymer können diesem entgegenwirken, z.B. Amphomer.

Kurzes, kräftiges Haar kann sich gut mit Gelen, Wachsen, Pasten, Cremes und Haarsprays in Form bringen und halten lassen.

Je länger die Haare sind, desto stärker kann der Wunsch des Nutzers sein, das Stylingprodukt vor dem zu Bett gehen aus dem Haar zu entfernen. Kurze Haare, die mit Wachsen, Pasten, Cremes, Gelen o.ä. gestylt wurden lassen sich zumeist schnell mit Wasser auswaschen und trocknen auch im Anschluss schnell (ggf. durch zur Hilfenahme eines Föhns). Längere Haare, die mit einem Stylingmousse in Form gebracht werden und/oder mit einem Haarspray fixiert werden, werden oftmals ausgebürstet. Zu diesem Zweck kann es hilfreich sein, wenn das angewendete fixierende Polymer leicht spröde und brüchig wird und sich z.B. durch eine Zuhilfenahme eines Kammes/einer Bürste entfernen lässt. Besagte Polymere wären bspw. PVP, PVP/VA, Amphomer, etc..

Nutzer mit afro-amerikanischem Haar können Produkte bevorzugen, die vor allem eines mit sich bringen: viel Pflege. Diese Zielgruppe kann das Gefühl haben, dass ihre Haare sehr trocken und spröde sind und viel Feuchtigkeit benötigen. Hier können insbesondere reichhaltige, cremige Texturen erwünscht sein, aber auch Ölsprays, die die Frisur des Nutzers gesund und gepflegt aussehen lassen. Konditionierende Polymere können helfen, das Haar in Form zu halten, ohne es steif und unflexibel wirken zu lassen. Reichhaltige Wachse und Emulsionen können den Haaren zusätzlich Pflege bereitstellen und können die Haare leicht beschweren, sodass die Haare nicht fliegen (Anti-Frizz). Was auf europäischem Haar also ein "no go" wäre, kann für afro-amerikanisches Haar gar nicht pflegend/beschwerend/fettig genug sein.

Nutzer mit geschädigtem, z.B. blondiertem, Haar können mehr Pflege in ihren Produkten benötigen als Personen mit gesundem ("virgin") Haar.

In verschiedenen Ausführungsbeispielen können zusätzlich zu den chemischen Eigenschaften oder stattdessen physikalische Eigenschaften der empfohlenen und nicht zu empfehlenden Pflege- und Stylingprodukte (DOs und DON'Ts, z.B. Viskositäten, Verdampfungseigenschaften, Klebrigkeit) ausschlaggebend oder einflussreich bezüglich dessen sein, ob ein Produkt für einen gegebenen Haartyp als empfehlenswert betrachtet wird oder nicht.

Je länger das Haar, desto geringer sollte eine Klebrigkeit von Formulierungen sein. Gerade Nutzer(innen) mit langem Haar wünschen sich häufig ein Stylingprodukt, welches hält und stylt, allerdings nicht stark klebt. Viele von ihnen können ein natürliches Haargefühl und -aussehen bevorzugen.

Neben Mousses und leichten Sprays oder Emulsionen zum Trockenföhnen können langhaarige Frauen auch häufig Haarspray verwenden, um ihre Frisur zu fixieren. Auch hier kann ein nicht stark klebendes Produkt bevorzugt sein oder werden. Zusätzlich kann es allerdings wünschenswert sein, dass das Haarspray feinst verteilt auf dem Haar ankommt, um oben besagten Effekt (eine festbetonierte/unnatürlich aussehende Frisur) zu vermeiden.

Auch Nutzer mit Locken können Wert darauflegen, die Locken zu unterstützen und natürlich aussehen zu lassen, und ihren Look nicht zu zementieren. Je dicker und kürzer das Haar, desto höher darf die Produktviskosität sein, da diese oftmals schon bei Stylen hilft, das Haar in Form zu bringen. Hier können sich, wie vorab bereits beschrieben, Pasten, Cremes, Gele mit einer hohen Viskosität eignen.

In verschiedenen Ausführungsbeispielen kann zusätzlich zu den chemischen Eigenschaften und/oder den physikalischen Eigenschaften eine Konfektion (auch als Anwendungsform, Aufbringungsform usw. bezeichnet) der empfohlenen und nicht zu empfehlenden Pflege- und Stylingprodukte (DOs und DON'Ts, z.B. eher Spray als Gel) ausschlaggebend oder einflussreich bezüglich dessen sein, ob ein Produkt für einen gegebenen Haartyp als empfehlenswert betrachtet wird oder nicht. Dies ist teilweise oben beschrieben.

In verschiedenen Ausführungsbeispielen kann die ermittelte Empfehlung genutzt werden für eine Herstellung eines optimalen/personalisiertem Haarbehandlungsprodukt, beispielsweise Stylingprodukts, z.B. als ein Auftrag für eine Herstellung eines optimalen/personalisierten Haarbehandlungsprodukt, beispielsweise Stylingprodukts.

Dies kann beispielsweise durch Aufrufen einer Internetseite eines Herstellers von optimalen/personalisierten Haarbehandlungsprodukten, wie beispielsweise Stylingprodukten, einzuleiten.

Bei dem optimalen/personalisierten Haarbehandlungsprodukt kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Mit Hilfe eines Produktkonfigurators kann beispielsweise die Auswahl chemisch und/oder physikalisch inkompatibler Inhaltsstoffe oder die Auswahl für den ermittelten Haarzustand ungeeigneter Inhaltsstoffe vermieden werden. Umgekehrt kann die Auswahl für den ermittelten Haarzustand geeigneter Inhaltsstoffe durch den Produktkonfigurator vorgegeben oder vorgeschlagen werden.

In verschiedenen alternativen Ausführungsbeispielen kann eine Nutzereingabe an einer Abgabevorrichtung zur optimierten Abgabe von Haarbehandlungsprodukt genutzt werden. Diese Abgabevorrichtung kann vorzugsweise bei einem Friseur oder an einem Verkaufspunkt ("point of sale", POS) von Haarbehandlungsmitteln vorhanden sein. Der Nutzer kann mithilfe einer digitalen Anzeige und eines Touchscreens seinen Haarzustand und ggf. die gewünschte Menge an Produkt auswählen. Hierzu kann eine gespeicherte Empfehlung bereits im Gerät einprogrammiert sein, beispielsweise in einer Datenbank abgelegt (d.h. gespeichert) sein. Derlei Empfehlungen können von Beipackzetteln von Haarcolorationen bekannt sein, auf welchen häufig vermerkt sein kann, dass z.B. Bei schulterlangem Haar zwei Colorationspackungen angewendet werden sollten. Mittels einer vorherigen individuellen Eingabe der Haarlänge des Nutzers kann so eine (Mengen- und) Produktempfehlung abgegeben werden. Diese kann vom Nutzer entweder bestätigt, erweitert oder verringert werden, wenn dieser beispielsweise selbst schon weiß, dass er tendenziell mehr/weniger Produkt anwendet, als auf Verpackungen normalerweise angegeben.

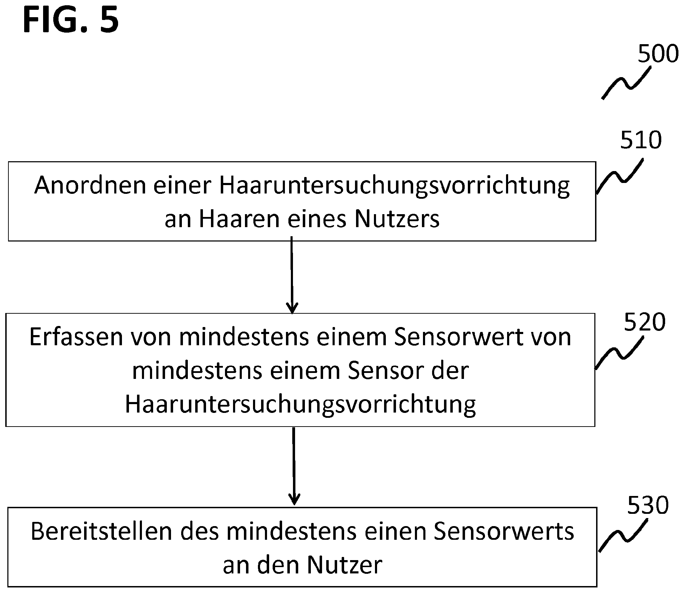

FIG. 5 zeigt ein Flussdiagramm 500 eines Verfahrens zum Bereitstellen einer Haarzustandsinformation gemäß verschiedenen Ausführungsbeispielen, wobei nur die Ausführungsbeispiele, bei denen mindestens zwei Sensorwerte erfasst werden, auch Ausführungsbeispiele der Erfindung sind.

Bei einem Verfahren zum Bereitstellen einer Haarzustandsinformation gemäß verschiedenen Ausführungsbeispielen kann eine Haaruntersuchungsvorrichtung gemäß verschiedenen Ausführungsbeispielen und/oder ein Haaruntersuchungssystem gemäß verschiedenen Ausführungsbeispielen genutzt werden.

Das Verfahren kann aufweisen ein Anordnen einer Haaruntersuchungsvorrichtung an Haaren eines Nutzers (z.B. die Haaruntersuchungsvorrichtung gemäß verschiedenen Ausführungsbeispielen, die beispielsweise Teil des Haaruntersuchungssystems gemäß verschiedenen Ausführungsbeispielen sein kann) (in 510), ein Erfassen von mindestens einem Sensorwert von mindestens einem Sensor der Haaruntersuchungsvorrichtung (in 520) und ein Bereitstellen des mindestens einen Sensorwerts an den Nutzer (in 530).

Für die oben beschriebenen Ermittlungen kann in verschiedenen Ausführungsbeispielen eine Programmierung, z.B. eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum Bereitstellen einer Haarzustandsinformation gemäß verschiedenen Ausführungsbeispielen ein Smartphone, ein Tablet oder ähnliches genutzt wird, kann die Software als eine App bereitgestellt sein.

In verschiedenen Ausführungsbeispielen, beispielsweise mittels Vergleichens der Sensorwerte mit bereitgestellten Referenzwerten, können z.B. ein geeignetes Haarpflegemittel, Haarstylingmittel, eine Haarbehandlungsempfehlung oder ähnliches für die Haare ermittelt werden.

In verschiedenen Ausführungsbeispielen kann die in der Haaruntersuchungsvorrichtung integrierte Schaltkreisvorrichtung und/oder eine externe Datenverarbeitungsvorrichtung, z.B. ein Smartphone, ein Tablet, ein Laptop, ein Smart Mirror, einer Smartwatch, ein iPad, oder ähnliches geeignet sein, um bei einem Ausführen des Verfahrens zum Bereitstellen einer Haarzustandsinformation, z.B. bei Ermittlungsvorgängen, z.B. mittels Vergleichens mit einer Datenbank/Referenzwerten o.ä., verwendet zu werden. In verschiedenen Ausführungsbeispielen braucht die Programmierung/Software nicht auf dem Smartphone, dem Tablet, dem Laptop usw. bereitgestellt zu werden. Es kann beispielsweise ausreichend sein, wenn die in die Haaruntersuchungsvorrichtung integrierte Schaltkreisvorrichtung und/oder das Smartphone o.ä. durch das Internet, mittels WLAN oder auf andere gängige Weise mit einer (z.B. einer weiteren) externen Datenverarbeitungsvorrichtung, z.B. einem Computer, beispielsweise einer Cloud, verbunden ist. In einem solchen Fall können die Berechnungen beispielsweise mittels der (weiteren) externen Datenverarbeitungsvorrichtung, z.B. mittels des Computers, ausgeführt werden, und das Ergebnis kann dem Smartphone/Tablet o.ä. und/oder der internen Schaltkreisvorrichtung bereitgestellt werden.

Weitere vorteilhafte Ausgestaltungen des Verfahrens ergeben sich aus der Beschreibung der Vorrichtung und umgekehrt.

## Patentansprüche

1. Haaruntersuchungsvorrichtung (100), aufweisend:
einen Vorrichtungskörper (1 00K), der auf mindestens einer Seite (100u) an seiner Oberfläche (100o) mit einer Haftschicht (110) versehen ist und der eingerichtet ist, mittels der Haftschicht lösbar im oder am Haar (220H) eines Nutzers (220) befestigt zu werden;
mindestens einen im oder am Vorrichtungskörper angeordneten Sensor (106) zum Erfassen von mindestens zwei Sensorwerten, wobei der mindestens eine Sensor eingerichtet ist, zwei oder mehrere Sensorwerte zu erfassen, wobei ein erster Sensorwert interne Daten betrifft und ein zweiter Sensorwert externe Daten betrifft, wobei die interne Daten ein kosmetisches Status oder ein Haarzustand betreffen und die externe Daten eine Außenumgebung des Nutzers betreffen; und
eine im oder am Vorrichtungskörper angeordnete elektronische Schaltkreisvorrichtung (104),
wobei die elektronische Schaltkreisvorrichtung mit dem mindestens einen Sensor gekoppelt ist zum Empfangen von den mindestens zwei Sensorwerten, und
wobei die elektronische Schaltkreisvorrichtung eingerichtet ist, den empfangenen mindestens zwei Sensorwerte dem Nutzer bereitzustellen,
wobei die elektronische Schaltkreisvorrichtung eingerichtet ist, basierend auf den ermittelten Sensorwerten eine Empfehlung zu ermitteln und dem Nutzer bereitzustellen,
wobei die Empfehlung mindestens eine Empfehlung ausgewählt aus der Gruppe bestehend aus Haarpflegeproduktempfehlungen, Haarstylingproduktempfehlungen und Haarbehandlungsempfehlungen umfasst.

2. Haaruntersuchungsvorrichtung (100), aufweisend:
einen Vorrichtungskörper (100K), der eingerichtet ist, im oder am Haar (220H) eines Nutzers (220) angeordnet zu werden, wobei der Vorrichtungskörper fadenförmig ist oder einen fadenförmigen Bereich (100F) aufweist;
mindestens einen Sensor (106) zum Erfassen von mindestens zwei Sensorwerten, wobei der Sensor oder mindestens einer der Sensoren in dem fadenförmigen Vorrichtungskörper oder dem fadenförmigen Bereich des Vorrichtungskörpers angeordnet ist, wobei der mindestens eine Sensor eingerichtet ist, zwei oder mehrere Sensorwerte zu erfassen, wobei ein erster Sensorwert interne Daten betrifft und ein zweiter Sensorwert externe Daten betrifft, wobei die interne Daten ein kosmetisches Status oder ein Haarzustand betreffen und die externe Daten eine Außenumgebung des Nutzers betreffen; und
eine im oder am Vorrichtungskörper angeordnete elektronische Schaltkreisvorrichtung (104),
wobei die elektronische Schaltkreisvorrichtung mit dem mindestens einen Sensor gekoppelt ist zum Empfangen von den mindestens zwei Sensorwerten; und
wobei die elektronische Schaltkreisvorrichtung eingerichtet ist, den empfangenen mindestens zwei Sensorwerte dem Nutzer bereitzustellen,
wobei die elektronische Schaltkreisvorrichtung eingerichtet ist, basierend auf dem ermittelten Sensorwert eine Empfehlung zu ermitteln und dem Nutzer bereitzustellen,
wobei die Empfehlung mindestens eine Empfehlung ausgewählt aus der Gruppe bestehend aus Haarpflegeproduktempfehlungen, Haarstylingproduktempfehlungen und Haarbehandlungsempfehlungen umfasst.

3. Haaruntersuchungsvorrichtung gemäß einem der Ansprüche 1 oder 2,
wobei der Sensor mindestens einen Sensor ausgewählt aus der Gruppe bestehend aus Luftfeuchtigkeitssensoren, Temperatursensoren, UV-Strahlungssensorenaufweist .

4. Haaruntersuchungsvorrichtung gemäß einem der Ansprüche 1 oder 2, wobei der Sensor mindestens einen Sensor ausgewählt aus der Gruppe bestehend aus:
Haardickesensoren, Haarfeuchtigkeitssensoren, Schädigungsgradsensoren, und Haar- und/oder Hautfettgehaltsensoren aufweist.

5. Haaruntersuchungsvorrichtung gemäß einem der Ansprüche 1 bis 4,
wobei der Sensor optisch und/oder physikalisch in Kontakt ist mit einer äußeren Oberfläche des Vorrichtungskörpers.

6. Haaruntersuchungsvorrichtung gemäß einem der Ansprüche 1 oder 3 bis 5, wobei die Haftschicht einen druckempfindlichen Klebstoff und/oder ein Klettmaterial aufweist.

7. Haaruntersuchungsvorrichtung gemäß einem der Ansprüche 1 bis 6,
wobei die elektronische Schaltkreisvorrichtung eine kabellose Datenaustauschvorrichtung aufweist.

8. Haaruntersuchungssystem (200, 200a, 200b), aufweisend:
eine Haaruntersuchungsvorrichtung (100) gemäß Anspruch 7; und
eine Anzeigevorrichtung (228),
wobei die mindestens eine Haaruntersuchungsvorrichtung eingerichtet ist, der Anzeigevorrichtung den Sensorwerten und/oder die Empfehlung mittels der Datenaustauschvorrichtung zu übermitteln.

9. Haaruntersuchungssystem gemäß Anspruch 8,
wobei die Anzeigevorrichtung einen Computerbildschirm, ein Smartphone, ein Tablet, eine Smartwatch, oder einen Laptop aufweist.

10. Verfahren zum Bereitstellen einer Haarzustandsinformation, aufweisend:
Anordnen einer Haaruntersuchungsvorrichtung (100) gemäß einem der Ansprüche 1 bis 7 oder einer Haaruntersuchungsvorrichtung eines Haaruntersuchungssystems (200, 200a, 200b) gemäß einem der Ansprüche 8 oder 9 an Haaren (220H) eines Nutzers (220);
Erfassen von mindestens zwei Sensorwerten des mindestens einen Sensors (106) der Haaruntersuchungsvorrichtung; und
Bereitstellen von den mindestens zwei Sensorwerten an den Nutzer,
Ermitteln mindestens einer Empfehlung basierend auf dem mindestens einen Sensorwert,
wobei die Empfehlung mindestens eine Empfehlung ausgewählt aus der Gruppe bestehend aus Haarpflegeproduktempfehlungen, Haarstylingproduktempfehlungen und Haarbehandlungsempfehlungen umfasst.

11. Verfahren gemäß Anspruch 10 ,
wobei das Anordnen der Haaruntersuchungsvorrichtung ein Anordnen für einen, verglichen mit einer für ein einzelnes Erfassen von den mindestens zwei Sensorwerten benötigten Zeitdauer, langen Zeitraum aufweist, und
wobei das Erfassen von den mindestens zwei Sensorwerten während des Zeitraums wiederholt oder kontinuierlich vorgenommen wird.

12. Verfahren gemäß einem der Ansprüche 10 oder 11, ferner aufweisend:
Übermitteln von den mindestens zwei Sensorwerten an eine externe Datenverarbeitungsvorrichtung (226); und
Empfangen der mindestens einen Empfehlung von der externen Datenverarbeitungsvorrichtung,
wobei das Ermitteln der mindestens einen Empfehlung basierend auf den mindestens zwei Sensorwerten mittels der externen Datenverarbeitungsvorrichtung erfolgt.

## Claims

1. A hair analyzing device (100), comprising:
a device body (100K) which is provided, on its surface (100o) on at least one side (100u), with an adhesive layer (110) and which is designed to be releasably fastened in or on the hair (220H) of a user (220) by means of the adhesive layer;
at least one sensor (106) which is arranged in or on the device body and is intended for detecting at least two sensor values, wherein the at least one sensor is designed to detect two or more sensor values, wherein a first sensor value relates to internal data and a second sensor value relates to external data, wherein the internal data relate to a cosmetic status or a hair condition and the external data relate to the external environment of the user; and
an electronic circuit device (104) arranged in or on the device body,
wherein the electronic circuit device is coupled to the at least one sensor in order to receive the at least two sensor values, and wherein the electronic circuit device is designed to provide the received at least two sensor values to the user, wherein the electronic circuit device is designed, on the basis of the determined sensor value, to determine a recommendation and to provide said recommendation to the user,
wherein the recommendation includes at least one recommendation selected from the group consisting of hair care product recommendations, hair styling product recommendations and hair treatment recommendations.

2. A hair analyzing device (100), comprising:
a device body (100K) which is designed to be arranged in or on the hair (220H) of a user (220), wherein the device body is filiform or comprises a filiform region (100F);
at least one sensor (106) for detecting at least two sensor values, wherein the sensor or at least one of the sensors is arranged in the filiform device body or the filiform region of the device body, wherein the at least one sensor is designed to detect two or more sensor values, wherein a first sensor value relates to internal data and a second sensor value relates to external data, wherein the internal data relates to a cosmetic status or a hair condition and the external data relates to the external environment of the user; and
an electronic circuit device (104) arranged in or on the device body,
wherein the electronic circuit device is coupled to the at least one sensor in order to receive the at least two sensor values; and wherein the electronic circuit device is designed to provide the received at least two sensor values to the user, wherein the electronic circuit device is designed, on the basis of the determined sensor value, to determine a recommendation and to provide said recommendation to the user, wherein the recommendation includes at least one recommendation selected from the group consisting of hair care product recommendations, hair styling product recommendations and hair treatment recommendations.

3. The hair analyzing device according to one of claims 1 or 2, wherein the sensor comprises at least one sensor selected from the group consisting of humidity sensors, temperature sensors and UV-radiation sensors.

4. The hair analyzing device according to one of claims 1 or 2, wherein the sensor comprises at least one sensor selected from the group consisting of: hair thickness sensors, hair moisture sensors, damage level sensors, and hair and/or skin fat content sensors.

5. The hair analyzing device according to one of claims 1 to 4, wherein the sensor is optically and/or physically in contact with an outer surface of the device body.

6. The hair analyzing device according to one of claims 1 or 3 to 5, wherein the adhesive layer comprises a pressure sensitive adhesive and/or a hook-and-loop material.

7. The hair analyzing device according to one of claims 1 to 6, wherein the electronic circuit device comprises a wireless data exchange device.

8. A hair analyzing system (200, 200a, 200b) comprising:
a hair analyzing device (100) according to claim 7; and
a display device (228),
wherein the at least one hair analyzing device is designed to transmit, to the display device, the sensor values and/or the recommendation by means of the data exchange device.

9. The hair analyzing system according to claim 8,
wherein the display device comprises a computer screen, a smartphone, a tablet, a smartwatch or a laptop.

10. A method for providing hair condition information, comprising:
arranging, on hair (220H) of a user (220), a hair analyzing device (100) according to one of claims 1 to 7 or a hair analyzing device of a hair analyzing system (200, 200a, 200b) according to one of claims 8 or 9;
detecting at least two sensor values of the at least one sensor (106) of the hair analyzing device; and
providing the at least two sensor values to the user;
determining at least one recommendation on the basis of the at least one sensor value,
wherein the recommendation includes at least one recommendation selected from the group consisting of hair care product recommendations, hair styling product recommendations and hair treatment recommendations.

11. The method according to claim 10,
wherein the arrangement of the hair analyzing device comprises arranging for a long period of time compared to a duration required for single detection of the at least two sensor values, and
wherein the detection of the at least two sensor values is performed repeatedly or continuously during the period of time.

12. The method according to one of claims 10 or 11, further comprising:
transmitting the at least two sensor values to an external data processing device (226); and
receiving the at least one recommendation from the external data processing device,
wherein the determination of the at least one recommendation on the basis of the at least two sensor values is carried out by means of the external data processing device.

## Revendications

1. Dispositif d'analyse des cheveux (100), présentant :
un corps de dispositif (100K) qui est pourvu d'une couche adhésive (110) sur sa surface (100o) sur au moins un côté (100u) et qui est conçu pour être fixé, au moyen de la couche adhésive, de manière amovible dans ou sur les cheveux (220H) d'un utilisateur (220) ;
au moins un capteur (106) disposé dans ou sur le corps de dispositif pour la détection d'au moins deux valeurs de capteur, l'au moins un capteur étant configuré pour détecter deux valeurs de capteur ou plus, une première valeur de capteur concernant des données internes et une seconde valeur de capteur concernant des données externes, les données internes concernant un état cosmétique ou un état des cheveux et les données externes concernant un environnement extérieur de l'utilisateur ; et
un dispositif de circuit électronique (104) disposé dans ou sur le corps de dispositif,
le dispositif de circuit électronique étant couplé à l'au moins un capteur pour la réception des au moins deux valeurs de capteur, et le dispositif de circuit électronique étant configuré pour fournir à l'utilisateur les au moins deux valeurs de capteur reçues, le dispositif de circuit électronique étant configuré pour, sur la base des valeurs de capteur déterminées, déterminer une recommandation et la fournir à l'utilisateur,
la recommandation comprenant au moins une recommandation choisie dans le groupe constitué de recommandations de produits de soins capillaires, recommandations de produits coiffants et recommandations de traitements capillaires.

2. Dispositif d'analyse des cheveux (100), présentant :
un corps de dispositif (100K) qui est conçu pour être disposé dans ou sur les cheveux (220H) d'un utilisateur (220), le corps de dispositif étant filiforme ou présentant une région filiforme (100F) ;
au moins un capteur (106) pour la détection d'au moins deux valeurs de capteur, le capteur ou au moins l'un des capteurs étant disposé dans le corps de dispositif filiforme ou la région filiforme du corps de dispositif, l'au moins un capteur étant configuré pour détecter deux valeurs de capteur ou plus, une première valeur de capteur concernant des données internes et une seconde valeur de capteur concernant des données externes, les données internes concernant un état cosmétique ou un état des cheveux et les données externes concernant un environnement extérieur de l'utilisateur ; et
un dispositif de circuit électronique (104) disposé dans ou sur le corps de dispositif,
le dispositif de circuit électronique étant couplé à l'au moins un capteur pour la réception des au moins deux valeurs de capteur ; et
le dispositif de circuit électronique étant configuré pour fournir à l'utilisateur les au moins deux valeurs de capteur reçues,
le dispositif de circuit électronique étant configuré pour, sur la base de la valeur de capteur déterminée, déterminer une recommandation et la fournir à l'utilisateur,
la recommandation comprenant au moins une recommandation choisie dans le groupe constitué de recommandations de produits de soins capillaires, recommandations de produits coiffants et recommandations de traitements capillaires.

3. Dispositif d'analyse des cheveux selon l'une des revendications 1 ou 2, le capteur présentant au moins un capteur choisi dans le groupe constitué de capteurs d'humidité de l'air, capteurs de température, capteurs de rayonnement UV.

4. Dispositif d'analyse des cheveux selon l'une des revendications 1 ou 2, le capteur présentant au moins un capteur choisi dans le groupe constitué de : capteurs d'épaisseur des cheveux, capteurs d'humidité des cheveux, capteurs de degré d'endommagement et capteurs de teneur en graisse des cheveux et/ou de la peau.

5. Dispositif d'analyse des cheveux selon l'une des revendications 1 à 4, le capteur étant en contact de manière optique et/ou physique avec une surface extérieure du corps de dispositif.

6. Dispositif d'analyse des cheveux selon l'une des revendications 1 ou 3 à 5, la couche adhésive présentant un adhésif sensible à la pression et/ou un matériau autoagrippant.

7. Dispositif d'analyse des cheveux selon l'une des revendications 1 à 6, le dispositif de circuit électronique présentant un dispositif d'échange de données sans fil.

8. Système d'analyse des cheveux (200, 200a, 200b), présentant :
un dispositif d'analyse des cheveux (100) selon la revendication 7 ; et
un dispositif d'affichage (228),
l'au moins un dispositif d'analyse des cheveux étant configuré pour transmettre au dispositif d'affichage les valeurs de capteur et/ou la recommandation au moyen du dispositif d'échange de données.

9. Système d'analyse des cheveux selon la revendication 8,
le dispositif d'affichage présentant un écran d'ordinateur, un mobile multifonction, une tablette, une montre intelligente ou un ordinateur portable.

10. Procédé permettant de fournir une information concernant l'état des cheveux, présentant :
la disposition d'un dispositif d'analyse des cheveux (100) selon l'une des revendications 1 à 7 ou d'un dispositif d'analyse des cheveux d'un système d'analyse des cheveux (200, 200a, 200b) selon l'une des revendications 8 ou 9 sur les cheveux (220H) d'un utilisateur (220) ;
la détection d'au moins deux valeurs de capteur de l'au moins un capteur (106) du dispositif d'analyse des cheveux ; et
la fourniture des au moins deux valeurs de capteur à l'utilisateur,
la détermination d'au moins une recommandation sur la base de l'au moins une valeur de capteur,
la recommandation comprenant au moins une recommandation choisie dans le groupe constitué de recommandations de produits de soins capillaires, recommandations de produits coiffants et recommandations de traitements capillaires.

11. Procédé selon la revendication 10,
la disposition du dispositif d'analyse des cheveux présentant une disposition pendant un laps de temps long comparé à une durée requise pour une seule détection des au moins deux valeurs de capteur, et
la détection des au moins deux valeurs de capteur étant effectuée de manière répétée ou continue pendant le laps de temps.

12. Procédé selon l'une des revendications 10 ou 11, présentant en outre :
la transmission des au moins deux valeurs de capteur à un dispositif de traitement de données externe (226) ; et
la réception de l'au moins une recommandation en provenance du dispositif de traitement de données externe,
la détermination de l'au moins une recommandation étant réalisée sur la base des au moins deux valeurs de capteur au moyen du dispositif de traitement de données externe.
